(19) Europäisches Patentamt
European Patent Office
Office européen des brevets

(11) **EP 3 571 997 B1**

(12) **EUROPÄISCHE PATENTSCHRIFT**

(45) Veröffentlichungstag und Bekanntmachung des Hinweises auf die Patenterteilung:
**23.11.2022 Patentblatt 2022/47**

(21) Anmeldenummer: **18212734.0**

(22) Anmeldetag: **14.12.2018**

(51) Internationale Patentklassifikation (IPC):
*A61B 6/00* (2006.01)   *A61B 5/00* (2006.01)
*A61B 5/107* (2006.01)   *G16H 30/40* (2018.01)
*G16H 50/30* (2018.01)   *G16H 50/50* (2018.01)
*A61B 6/03* (2006.01)   *G06T 11/00* (2006.01)

(52) Gemeinsame Patentklassifikation (CPC):
**A61B 6/488; A61B 5/0077; A61B 5/1073;
A61B 6/5217; A61B 6/5294; A61B 6/544;
A61B 6/545; G06T 11/008; G16H 30/40;
G16H 50/30; G16H 50/50;** A61B 5/7264;
A61B 6/032

(54) **VERFAHREN UND VORRICHTUNG ZUM BESTIMMEN EINES PATIENTENGE-WICHTS UND/ODER EINES BODY-MASS-INDEX**

METHOD AND DEVICE FOR DETERMINING THE WEIGHT OF A PATIENT AND/OR A BODY MASS INDEX

PROCÉDÉ ET DISPOSITIF DE DÉTERMINATION D'UN POIDS DE PATIENT ET / OU D'UN INDICE DE MASSE CORPORELLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.05.2018 EP 18173815**

(43) Veröffentlichungstag der Anmeldung:
**27.11.2019 Patentblatt 2019/48**

(73) Patentinhaber: **Siemens Healthcare GmbH
91052 Erlangen (DE)**

(72) Erfinder:
• **Prokein, Andreas
91088 Bubenreuth (DE)**
• **Raupach, Rainer
91336 Heroldsbach (DE)**
• **Wimmer, Andreas
91301 Forchheim (DE)**

(56) Entgegenhaltungen:
US-A1- 2006 067 461    US-A1- 2014 270 053
US-A1- 2016 262 713    US-A1- 2016 262 714
US-A1- 2017 249 423

**Beschreibung**

Die Erfindung betrifft ein Verfahren und eine Vorrichtung zur Bestimmung eines Patientengewichts und/oder eines Body-Mass-Index, sowie ein Computerprogrammprodukt mit einem Computerprogramm und ein computerlesbares Medium, auf welchem von einer Recheneinheit einlesbare und ausführbare Programmabschnitte gespeichert sind, zur Ausführung des erfindungsgemäßen Verfahrens.

Für bestimmte Zwecke, z.B. eine patientenspezifische CT-Kontrastmittelgabe, ist die Kenntnis des Patientengewichts erforderlich. Ziel der Erfindung ist eine Bestimmung des Patientengewichts bzw. eines Body-Mass-Index, ohne das Patientengewicht direkt mit Hilfe einer mechanischen Waage messen zu müssen.

Die klassische Art der Gewichtsmessung ist die mit einer Waage, auf die sich der Patient stellt/setzt/legt. Dies stellt aber einen zusätzlichen Schritt im Workflow dar und beansprucht daher zusätzliche Zeit. Natürlich könnte die Waage zur Vermeidung eines zusätzlichen Arbeitsschrittes auch direkt in den CT-Tisch eingebaut werden. Dies führt aber zu erhöhten Hardware-Kosten. Es gibt auch Ansätze, durch die gewichtsabhängige Trägheit des Tisches bei Horizontalbeschleunigung das Gewicht abzuleiten, z.B. über die Messung des für eine bestimmte Beschleunigung notwendigen Motorstroms.

Ferner wäre es naheliegend, aus einem mithilfe einer optischen 3D-Kamera bestimmte Oberflächenmodell des Körpers über das Volumen und mit einer angenommenen mittleren Dichte das Patientengewicht zu schätzen. Problematisch in einer ausschließlich oberflächenbasierten Gewichtsabschätzung ist es jedoch, dass sich, beispielsweise aufgrund eines unterschiedlichen Körperfettanteils, bei gleichem Patientenvolumen deutlich unterschiedliche Gewichte ergeben können.

Dokument US 2016/262714 A1 offenbart, dass das Patienten-Gesamtgewicht nicht nur unter Verwendung des Oberflächenmodells ermittelt wird, sondern dass zusätzlich auch die (Massen-) Dichteinformation berücksichtigt wird. Dafür werden Daten aus einer Referenzdatenbank verwendet, die mittels einer Röntgenaufnahme für einen Referenzpatienten ermittelt wurden, der eine vergleichbare Statur aufweist.

Der Erfindung liegt somit die Aufgabe zugrunde, ein Verfahren anzugeben, dass ein Gewicht oder einen Body-Mass-Index eines Patienten mit geringem Aufwand und zugleich mit guter Genauigkeit ermitteln kann.

Die Aufgabe wird erfindungsgemäß durch ein Verfahren zum Bestimmen eines Patientengewichts und/oder eines Body-Mass-Index eines Patienten gelöst, das die folgenden Schritte aufweist:

- Erfassen von Bilddaten mit einer Tiefeninformation des Patienten,
- Basierend auf den Bilddaten, Erzeugen eines Oberflächenmodells des Patienten,
- Ermitteln von einer (Massen-)Dichteinformation aus einer Röntgenmessung des Patienten oder Röntgenschwächungsinformation von zumindest einem Teil des Patienten, und
- Ermitteln eines Patienten-Gesamtgewichts und/oder des Body-Mass-Index des Patienten unter Verwendung des Oberflächenmodells und der, insbesondere partiellen, Dichteinformation oder Röntgenschwächungsinformation.

Es wird somit vorgeschlagen, im Rahmen der Berechnung eines Patientengewichts oder eines Body-Mass-Index zusätzlich zu einem Oberflächenmodell des Patienten, aus dem insbesondere ein Patientenvolumen bekannt ist, Informationen über die Dichte bzw. Massendichte zumindest eines Teils des Patienten bzw. die Röntgenschwächung in zumindest einem Teil des Patienten zu berücksichtigen. Die Worte Patientengewicht und Patienten-Gesamtgewicht sowie die Worte Dichteinformation, Massendichteninformation und (Massen-)Dichteinformation werden hierbei austauschbar verwendet. Dadurch, dass zusätzliche Informationen über die Dichte des Patienten berücksichtigt werden, kann die Genauigkeit des ermittelten Patientengewichts bzw. des Body-Mass-Index gegenüber einer reinen Berücksichtigung des Patientenvolumens deutlich verbessert werden, da beispielsweise Auswirkungen von unterschiedlichen Körperfettgehalten zumindest näherungsweise berücksichtigt werden können. Hierbei kann die Dichteinformation ausschließlich eine mittlere Dichte des Patienten bzw. eines Teil des Patienten beschreiben. Vorzugsweise weist die Dichteinformation jedoch eine Ortsauflösung auf. Die Dichteinformation kann prinzipiell dreidimensional sein, also für einzelne Volumenabschnitte bzw. Voxel jeweils Informationen bezüglich der dortigen Dichte enthalten. Wie später noch genauer erläutert werden wird, kann eine ortsaufgelöste Dichteinformation jedoch besonders einfach erfasst werden, wenn Linienintegrale der Dichte entlang mehrerer Strahlen durch den Patientenkörper bestimmt werden. Da, wie erläutert, zur Ermittlung des Patienten-Gesamtgewichts bzw. des Body-Mass-Index des Patienten nicht notwendigerweise Dichteinformationen bezüglich des gesamten Volumen des Patienten zur Verfügung stehen, werden im Rahmen der Ermittlung typischerweise partielle Dichteinformationen, also insbesondere Dichteinformationen, die nur ein Teilvolumen des Patienten betreffen, berücksichtigt. Es ist jedoch selbstverständlich auch möglich, Dichteinformationen bezüglich des gesamten Volumens des Patienten zu berücksichtigen, falls diese vorliegen.

Wie später noch genauer erläutert werden kann, kann eine Röntgenschwächungsinformation, die beispielsweise aus Röntgenprojektionsdaten oder aus einer CT-Rekonstruktion anhand mehrerer Röntgenprojektionen ermittelt werden kann, ein Maß für die Massendichte im Patienten sein und somit auch als Sonderfall einer (Massen-)Dichteinformation betrachtet werden. In einer engeren Auslegung des Begriffs (Massen- ) tDichteinformation könnte auch gefordert werden, dass diese explizit eine Massendichte bzw. z.B. ein Linienintegral der Massedichte beschreibt. Eine derartige (Massen- ) Dichteinformation wird, wie später noch genauer erläutert werden wird, aus einer Röntgenschwächungsinformation berechnet.

Unter dem Erfassen von Bilddaten ist vorliegend einerseits eine Ermittlung der Bilddaten anhand einer entsprechenden Sensorik zu verstehen. Unter dem Erfassen von Bilddaten kann jedoch andererseits auch verstanden werden, dass diese von einer externen Einrichtung bereitgestellt und beispielsweise von einem Server oder einer anderen Verarbeitungseinrichtung über eine Kommunikationsverbindung erfasst werden. Ebenso kann das Ermitteln der Dichteinformation bzw. Röntgenschwächungsinformation ein sensorisches Erfassen von Eingangsdaten, aus denen diese ermittelt werden, umfassen. Es ist jedoch auch möglich, dass die Dichteinformation aus separat bereitgestellten Eingangsdaten ermittelt wird, die beispielsweise von einer Dritteinrichtung über eine Kommunikationsverbindung bereitgestellt werden können.

Die Bilddaten mit einer Tiefeninformation können insbesondere 3D-Bilddaten sein.

Die Bilddaten mit einer Tiefeninformation können insbesondere von einer 3D-Bildkamera erfasst worden sein. Insbesondere können die Bilddaten mit einer optischen 3D-Kamera erfasst werden oder erfasst worden sein. Wie eingangs erläutert, kann das erfindungsgemäße Verfahren insbesondere im Rahmen der Computertomographie oder bei der Berücksichtigung eines Patientengewichts bzw. Body-Mass-Index in anderen medizinischen Untersuchungsmodalitäten relevant sein. Bei diesen ist es häufig ohnehin erforderlich, eine korrekte Patientenpositionierung sicherzustellen bzw. bei der Planung der Messdatenerfassung zu berücksichtigen. Daher sind entsprechende 3D-Kameras bei neueren Computertomographiesystemen bzw. in anderen medizinischen Untersuchungseinrichtungen häufig ohnehin vorhanden.

Das Oberflächenmodell kann ein Patientenmodell, auch Avatar genannt, sein oder als Teil hiervon bereitgestellt werden. Da die Bilddaten Tiefeninformationen umfassen, bilden diese die Oberfläche des Patienten unmittelbar ab, so dass ein Oberflächenmodell im einfachsten Fall durch eine Segmentierung des Patienten in den Bilddaten erstellt werden kann. Besonders vorteilhaft kann das Oberflächenmodell jedoch mithilfe einer Machine-learning-basierten Methode aus den Bilddaten extrahiert werden. Die Extraktion eines Oberflächenmodells aus Bilddaten mit einer Tiefeninformation mithilfe von Machine-Learning-basierten Methoden ist prinzipiell im Stand der Technik bekannt und soll daher nicht detailliert erläutert werden.

Die (Massen-) Dichteinformation wird aus einer Röntgenmessung des Patienten ermittelt. Insbesondere kann die (Massen-)Dichteinformation aus Röntgenprojektionsdaten, insbesondere aus Röntgenprojektionsdaten einer Topogrammaufnahme, oder aus rekonstruierten Bilddaten eines nativen CT-Scans ermittelt werden.

Entsprechend kann die Röntgenschwächungsinformation aus einer Röntgenmessung des Patienten ermittelt werden. Insbesondere kann die Röntgenschwächungsinformation Röntgenprojektionsdaten, insbesondere Röntgenprojektionsdaten einer Topogrammaufnahme, oder rekonstruierten Bilddaten eines nativen CT-Scans beschreiben. Die (Massen-)Dichteinformation kann aus der Röntgenschwächungsinformation ermittelt werden.

Die Röntgenprojektionsdaten können insbesondere bei einer Topogrammaufnahme erzeugt werden, wie sie zur Vorbereitung von tomographischen Untersuchungen an einem Computertomographiegerät (CT-Gerät) durchgeführt werden. Hierbei werden eine oder mehrere separate Projektionsaufnahmen, also zweidimensionale Bildaufnahmen, durchgeführt, die zur Untersuchungsplanung genutzt werden. Beispielsweise kann ein Topogramm genutzt werden, um den eigentlichen Scanbereich für eine CT-Untersuchung, also insbesondere für eine dreidimensionale Datenaufnahme, exakt zu definieren. Da entsprechende Röntgenprojektionsdaten bei einem regulären Ablauf einer CT-Untersuchung daher ohnehin erfasst werden, muss keine zusätzliche Strahlung zum Zweck der Gewichtsbestimmung mithilfe dieser Daten appliziert werden. Die (Massen)Dichteinformation bzw. Röntgenschwächungsinformation kann somit anhand von Eingangsdaten ermittelt werden, die ohnehin vorhanden sind und im Rahmen des beschriebenen Verfahrens nur für eine weitere Funktion genutzt werden. Somit ist das erfindungsgemäße Verfahren besonders einfach, ohne zusätzliche Belastung des Patienten und im Wesentlichen ohne zusätzlichen Zeitaufwand einsetzbar.

In einigen Anwendungsfällen kann bei der Anwendung des erfindungsgemäßen Verfahrens bereits ein rekonstruierter Bilddatensatz, insbesondere ein rekonstruierter Bilddatensatz eines nativen CT-Scans, also eines Scans ohne Kontrastmittel, vorliegen, der dann genutzt werden kann, um die Dichteinformation zu ermitteln, bzw. der als Röntgenschwächungsinformation genutzt werden kann. Beispielsweise kann die erfindungsgemäße Bestimmung des Patientengewichts bzw. des Body-Mass-Index genutzt werden, um eine zu nutzende Kontrastmittelmenge zu bestimmen. In Kontrastmittel nutzenden Computertomographieverfahren werden häufig Maskenbilder bzw. entsprechende rekonstruierte Maskenbilddatensätze genutzt, die ohne vorangehende Kontrastmittelgabe erfasst wurden. Es können daher bereits rekonstruierte Bilddaten eines nativen CT-Scans vorliegen, wenn das Patientengewicht bzw. der Body-Mass-Index zur Parametrisierung der Kontrastmittelgabe bestimmt werden soll. Es liegt somit eine räumliche Schwächungsverteilung vor, aus der die Dichteverteilung ermittelt werden kann. Im einfachsten Fall kann ein Mapping von Schwächungswerten auf Massedichte-werte erfolgen. Dies ist prinzipiell aus der Strahlentherapieplanung bekannt. Alternativ können indirekt Massedichteverteilungen mithilfe von Mehrmaterial-Zerlegungen rekonstruiert werden. Dies ist beispielsweise aus der Druckschrift DE 10 2016 09674 A1 bekannt. Werden Mehrenergie-Topogramme bzw. Mehrenergie-CT-Aufnahmen genutzt, die insbesondere mithilfe von Photonen-zählenden Detektoren erfasst werden können, sind zudem Verfahren bekannt, um auf Basis von Materialzerlegungen Massedichte-Linienintegrale bzw. räumliche Massedichte-Verteilungen zu berechnen, beispielsweise aus den folgenden Artikeln: R. E. Alvarez and A. Macovski, "Energy-selective reconstructions in X-ray computerized tomography," Phys.Med.Biol., 21 (5), pp.733-744, 1976, Williamson et.al., "On two-parametermodels of photon cross sections: Application to dual-energy CT imaging," Med.Phys. 33 (11), pp.4115-4129, 2006.

Bevorzugt werden die Röntgenprojektionsdaten durch eine Strahlenaufhärtungskorrektur korrigiert, wobei die (Massen) Dichteinformation aus den derart korrigierten Röntgenprojektionsdaten ermittelt wird. Alternativ kann eine Strahlenaufhärtungskorrektur auch genutzt werden, um eine Röntgenschwächungsinformation aus den Röntgenprojektionsdaten zu ermitteln, die dann zur Berechnung des Patienten-Gesamtgewichts bzw. des Body-Mass-Index verwendet wird. Röntgenprojektionsdaten definieren typischerweise eine Signalabschwächung in den einzelnen Bildpunkten bei bekanntem eingestrahlten Röntgenspektrum. Da die lokale Absorption bzw. Streuung jedoch von der Frequenz bzw. Energie der angestrahlten Röntgenstrahlung abhängt, resultiert zunächst für die gemessenen Linienintegrale $L_\mu$ der Abschwächung das folgende Linienintegral:

$$L_\mu = -\ln(I/I_0) = -\ln\left(\int I(E)\exp\left(-\int \mu(E,\vec{r})d\vec{r}\right)dE \Big/ \int I(E)dE\right)$$

Hierbei ist $I_0$ die eingestrahlte Intensität der Röntgenstrahlung und I die erfasste Intensität der Röntgenstrahlung. I(E) ist die energieabhängige Intensitätsverteilung der eingestrahlten Röntgenstrahlung und $\mu(E,r)$ ist der energieabhängige Absorptionskoeffizient an der Position r.

Es ist bekannt, dass sich das Spektrum von Röntgenstrahlung beim Durchdringen von Materie mit zunehmender Durchdringungstiefe zu höheren Energien hin verschiebt, weil die höherenergetischen Photonen weniger stark gestreut werden. Dieser Effekt führt bei einer nichtenergieaufgelösten Erfassung der Intensität der Röntgenstrahlung im Rahmen der Bildgebung potentiell zu Messartefakten. Es wurde erkannt, dass auch die Dichteinformation durch diesen Effekt verfälscht werden kann, wenn sie unmittelbar aus den Röntgenprojektionsdaten berechnet wird. Daher wird vorgeschlagen, die Dichteinformation anhand korrigierter Röntgenprojektionsdaten zu ermitteln, die durch Anwenden einer Strahlenaufhärtungskorrektur auf die Röntgenprojektionsdaten gewonnen werden. Die korrigierten Röntgenprojektionsdaten $L_\mu'$ werden hierbei als Funktion f(x) der Röntgenprojektionsdaten $L_\mu$ ermittelt:

$$L_\mu' = f(L_\mu) \, ,$$

wobei die Funktion f(x) vorzugsweise ein Polynom ist, also in der Form $f(x) = \sum_k a_k x^k$ dargestellt werden kann.

Die Koeffizienten $a_k$ können ermittelt werden, indem Referenzmessungen an Objekten mit bekannter Verteilung eines energieunabhängigen Absorptionskoeffizienten bei einem bestimmten eingestrahlten Röntgenspektrum durchgeführt werden, insbesondere Phantommessungen. Alternativ oder ergänzend können Simulationen der Röntgenbildgebung genutzt werden. Die Koeffizienten $a_k$ werden dann so gewählt, dass die folgende Bedingung erfüllt ist:

$$L_\mu' \approx \int \mu'(\vec{r})d\vec{r}$$

Hierbei können die energieunabhängigen Absorptionskoeffizienten $\mu'$ beispielsweise berechnet werden, in dem die energieabhängigen Absorptionskoeffizienten anhand des bekannten Spektrums der eingestrahlten Röntgenstrahlung gewichtet werden. Hierbei kann im Rahmen der Referenzmessung insbesondere Wasser zur Absorption genutzt werden, da angenommen werden kann, dass alle im Körper vorkommenden Materialien in führender Ordnung wasserähnlich sind.

Aus den Röntgenprojektionsdaten oder den korrigierten Röntgenprojektionsdaten kann unter Annahme gleichen durchstrahlten Materials, wobei insbesondere eine Durchstrahlung von Wasser angenommen wird, für jeden Bildpunkt der Röntgenprojektionsdaten eine integrierte Dichte als (Massen-)Dichteinformation berechnet werden. Somit werden die unterschiedlich starken Schwächungen näherungsweise als reine Dichteunterschiede betrachtet, das heißt es gilt:

$$L'_{\mu} \approx \int \mu'(\bar{r})d\bar{r} \approx \mu_{H_2O} \int \rho(\bar{r}) / \rho_{H_2O}(\bar{r})d\bar{r} = \mu_{H_2O} / \rho_{H_2O}(\bar{r}) \underbrace{\int \rho(\bar{r})d\bar{r}}_{=L_\rho} \quad .$$

Hierbei sind $\mu_{H2O}$ der Schwächungskoeffizient von Wasser und p $H_2O$ die Dichte von Wasser. Die Röntgenprojektionsdaten $L_{\mu}$ bzw. die korrigierten Röntgenprojektionsdaten $L_{\mu}'$ sind somit mit einem bekannten Proportionalitätsfaktor proportional zu einem Integral entlang des jeweiligen Röntgenstrahls über die Dichte p(r) des durchstrahlten Patienten an der jeweiligen Position r. Somit lassen sich als (Massen-)Dichteinformation diese Dichteintegrale $L_\rho$ angeben:

$$L_\rho \approx L'_\mu \cdot \rho_{H_2O} / \mu_{H_2O} = \rho_{H_2O} / \mu_{H_2O} \cdot f(L_\mu) \quad .$$

Da die (Massen-)Dichteinformation näherungsweise mit bekanntem Proportionalitätsfaktor zu den Röntgenprojektionsdaten, also zu einer Röntgenschwächungsinformation, proportional ist, kann auch die Röntgenschwächungsinformation selbst als eine (Masse-)Dichteinformation betrachtet werden. Alternativ kann die (Massen-)Dichteinformation wie obig erläutert in einem Zwischenschritt aus der Röntgenschwächungsinformation ermittelt werden, um anschließend die (Massen-)Dichteinformation zur Bestimmung des Patienten-Gesamtgewichts bzw. des Body-Mass-Index heranzuziehen.

In der Erfindung kann der Schritt des Ermittelns unter Anwendung eines Machine-Learning-Verfahrens, insbesondere unter Anwendung eines trainierten Algorithmus, erfolgen. Hierbei kann insbesondere ein trainiertes neuronales Netz bzw. ein entsprechend in einem neuronalen Netz trainierter Algorithmus zur Anwendung kommen.

Vorzugsweise erfolgt die Ermittlung des Patienten-Gesamtgewichts und/oder des Body-Mass-Index mithilfe eines Maschinen-Learning-basierten Ansatzes unter Verwendung des Oberflächenmodells und der (Massen-)Dichteinformation oder Röntgenschwächungsinformation als Eingangsdaten. Der Maschinen-Learning-basierte Ansatz kann hierbei dem obig genannten Maschinen-Learning-Verfahren entsprechen. Beispielsweise kann ein neuronales Netz, insbesondere ein convolutional neural network, trainiert werden, um das Patienten-Gesamtgewicht und/oder den Body-Mass-Index aus dem Oberflächenmodell und der Dichteinformation bzw. Röntgenschwächungsinformation als Eingangsdaten zu ermitteln.

Algorithmen, beispielsweise neuronale Netze, die im Rahmen eines Maschinenlernens trainiert werden, weisen eine Vielzahl von Parametern auf, die im Rahmen eines Lernprozesses geeignet für eine spätere Verwendung gewählt werden. Bei einem neuronalen Netz kann es sich bei diesen Parametern beispielsweise um die Eingangsgewichte der einzelnen Neuronen handeln. Ein im erfindungsgemäßen Verfahren zur Ermittlung des Patienten-Gesamtgewichts bzw. des Body-Mass-Index genutzter Algorithmus kann insbesondere im Rahmen eines überwachten Lernens trainiert werden. Hierbei werden eine Vielzahl von Trainingsdatensätzen bereitgestellt, die neben den jeweiligen Eingangsdaten, also dem Oberflächenmodell und der Dichteinformation bzw. Röntgenschwächungsinformation, jeweils die für diese Eingangsdaten gewünschten Ausgangsdaten, also das jeweilige Patienten-Gesamtgewicht bzw. den jeweiligen Body-Mass-Index, umfassen. Die gewünschten Ausgangsdaten für die Trainingsdatensätze können beispielsweise für den entsprechenden Patienten, an dem dieser Trainingsdatensatz ermittelt wurde, auf andere Weise ermittelt werden. Beispielsweise kann der Patient durch eine Waage gewogen werden, um das Patienten-Gesamtgewicht zu ermitteln und/oder es kann zusätzlich eine Größe gemessen werden, um aus dieser und seinem Gewicht den Body-Mass-Index zu bestimmen. Es können jedoch auch beliebig andere Ansätze zur Ermittlung des Patienten-Gesamtgewichts bzw. des Body-Mass-Index genutzt werden, um Ausgangsdaten für die Trainingsdatensätze bereitzustellen.

Im Rahmen des Trainings des Algorithmus kann dieser zunächst zufällig oder auf andere Weise parametrisiert sein. Der vorläufig parametrisierte Algorithmus wird auf die Eingangsdaten von zumindest Teilen der Trainingsdatensätze angewandt und die resultierenden Ausgaben des Algorithmus werden mit den im entsprechenden Trainingsdatensatz hinterlegten Sollwerten verglichen, um zu erkennen, inwieweit eine Parametrisierung des Algorithmus verändert werden sollte. Beispielsweise kann zum Training eines neuronalen Netzes der bekannte Ansatz der Backpropagation of Error genutzt werden.

Neben dem erfindungsgemäßen Verfahren betrifft die Erfindung eine Vorrichtung zum Bestimmen eines Patientengewichts, aufweisend

- eine Schnittstelle zum Erfassen von Bilddaten mit einer Tiefeninformation des Patienten;
- eine Erzeugungseinheit zum Erzeugen eines Oberflächenmodells des Patienten basierend auf den Bilddaten;
- eine Schnittstelle zum Erfassen von einer (Massen-) Dichteinformation aus einer Röntgenmessung des Patienten oder Röntgenschwächungsinformation von zumindest einem Teil des Patienten,
- eine Berechnungseinheit zum Ermitteln eines Patienten-Gesamtgewichts und/oder des Body-Mass-Index des Patienten unter Verwendung des Oberflächenmodells und der Massen-Dichteinformation oder Röntgenschwächungsinformation von zumindest einem Teil des Patienten, und

- eine Ausgabeeinheit zum Ausgeben des ermittelten Patientengewichts und/oder des Body-Mass-Index des Patienten.

Bei der Vorrichtung kann es sich um einen Teil einer medizinischen Bildgebungseinrichtung, beispielsweise eines Computertomographen, handeln. Die Vorrichtung kann alternativ eine separat von einer medizinischen Bildgebungseinrichtung ausgebildete Steuereinrichtung zur Steuerung der Bildgebungseinrichtung, beispielsweise ein Messplatzcomputer, sein. Es ist jedoch auch möglich, dass die Vorrichtung ausschließlich zur Auswertung der Messdaten dient. Beispielsweise kann die Vorrichtung ein Arbeitsplatzrechner sein, der zur Auswertung von Messdaten dient und nicht unmittelbar in den Betrieb einer medizinischen Bildgebungseinrichtung eingreifen kann. Es ist auch möglich, dass die Vorrichtung ein Server ist oder als Cloud von mehreren Recheneinrichtungen implementiert ist, der oder die beispielsweise die Bilddaten und die (Massen-)Dichteinformation bzw. eine Information, aus der diese ermittelt werden kann, über ein Netzwerk empfangen kann. Das ermittelte Patienten-Gesamtgewicht bzw. der ermittelte Body-Mass-Index kann dann lokal weiterverwertet werden, zur weiteren Benutzung über das oder ein anderes Netzwerk bereitgestellt werden oder beispielsweise zurück zur medizinischen Bildgebungseinrichtung übertragen werden, um dort beispielsweise eine Kontrastmittelgabe zu steuern oder einem Benutzer eine diese Steuerung betreffende Informationen bereitzustellen. Die Ausgabeeinheit kann zur Ausgabe an einen Benutzer oder an eine weitere Komponente oder Einrichtung dienen.

Die Vorrichtung kann weitergebildet werden, wie es vorangehend zum erfindungsgemäßen Verfahren erläutert wurde. Insbesondere kann die Berechnungseinheit ein Korrekturmittel umfassen, um die erläuterte Strahlenaufhärtungskorrektur durchzuführen und/oder sie kann ein Dichteberechnungsmittel umfassen, um die (Massen-)Dichteinformation wie erläutert aus den Röntgenprojektionsdaten oder den korrigierten Röntgenprojektionsdaten zu berechnen. Die beschriebenen Einheiten bzw. Mittel der Vorrichtung können durch einen jeweiligen oder gemeinsamen Prozessor, insbesondere durch entsprechende Programmmittel, FPGA, ASIC oder andere Komponenten implementiert sein.

Die Erfindung betrifft ferner ein Computerprogrammprodukt mit einem Computerprogramm, welches direkt in eine Speichereinrichtung einer Steuereinrichtung oder einer Rechenvorrichtung ladbar ist, mit Programmabschnitten, um alle Schritte des Verfahrens der Erfindung auszuführen, wenn das Computerprogramm in der Steuereinrichtung oder der Rechenvorrichtung ausgeführt wird. Wie bereits erläutert ist hierbei bezüglich des Erfassens der Bilddaten bzw. des Ermittelns der (Massen-) tDichteinformation bzw. Röntgenschwächungsinformation prinzipiell ausreichend, wenn diese Informationen von einer externen Einrichtung oder einem weiteren Computerprogramm bereitgestellt werden. Das Computerprogramm kann jedoch auch Programmabschnitte umfassen, die die (Massen-)Dichteinformation beispielsweise, wie vorangehend erläutert, aus Röntgendaten ermitteln und/oder die ein Bilderfassungsmittel zur Erfassung der Bilddaten und/oder eine Röntgeneinrichtung zur Ermittlung entsprechender Röntgendaten bzw. der Röntgenschwächungsinformation ansteuern.

Die Erfindung betrifft ferner ein computerlesbares Medium, auf welchem von einer Recheneinheit einlesbare und ausführbare Programmabschnitte gespeichert sind, um alle Schritte des Verfahrens der Erfindung auszuführen, wenn die Programmabschnitte von der Recheneinheit ausgeführt werden. Die Programmabschnitte können insbesondere das obig erläuterte Computerprogramm bilden. Die Recheneinheit kann die Steuereinrichtung oder Rechenvorrichtung oder einen Teil hiervon bilden.

Die Erfindung betrifft zudem ein Verfahren zum Training eines Algorithmus zur Ermittlung eines Patienten-Gesamtgewichts und/oder eines Body-Mass-Index eines Patienten unter Verwendung eines Oberflächenmodells des Patienten und einer (Massen-) Dichteinformation bzw. Röntgenschwächungsinformation von zumindest einem Teil des Patienten. Der Algorithmus kann durch ein überwachtes Lernen anhand von mehreren Trainingsdatensätzen trainiert werden. Als Algorithmus kann insbesondere ein neuronales Netz trainiert werden. Details zum Training des Algorithmus und zur Bereitstellung der Trainingsdatensätze wurden bereits vorangehend erläutert.

Die Erfindung betrifft zudem einen durch das beschriebene Verfahren trainierte Algorithmus zur Ermittlung des Patienten-Gesamtgewichts und/oder des Body-Mass-Index eines Patienten sowie einen Datenträger, der diesen Algorithmus oder Parameter, die im Rahmen des Trainings ermittelt wurden, speichert.

Weitere Vorteile und Einzelheiten der Erfindung ergeben sich aus den folgenden Ausführungsbeispielen sowie den zugehörigen Zeichnungen. Diese zeigen schematisch:

Fig. 1    eine Darstellung eines Ausführungsbeispiels des erfindungsgemäßen Verfahrens,

Fig. 2    eine Darstellung eines Ausführungsbeispiels einer erfindungsgemäßen Vorrichtung,

Fig. 3    den Ablauf eines weiteren Ausführungsbeispiels des erfindungsgemäßen Verfahrens, und

Fig. 4    ein Ausführungsbeispiel zum Training eines Algorithmus, der zur Ermittlung eines Patienten-Gesamtgewichts und/oder eines Body-Mass-Index eines Patienten aus einem Oberflächenmodell und einer Dichteinformation bzw. Röntgenschwächungsinformation verwendet werden kann.

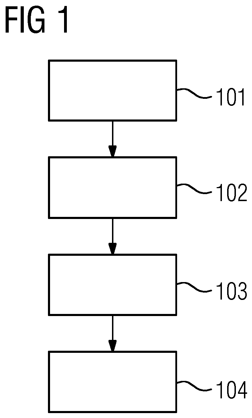

Fig. 1 zeigt ein Ablaufdiagramm eines Ausführungsbeispiels eines Verfahrens zur Ermittlung eines Patienten-Gesamtgewichts und/oder eines Body-Mass-Index eines Patienten. In den Ausführungsbeispielen erfolgt zur Vermeidung von unnötigen Wiederholungen die Ermittlung stets anhand von (Massen-)Dichteinformationen, die insbesondere in Abhängigkeit von Röntgendaten ermittelt werden können. Alternativ wäre es auch möglich, Röntgenschwächungsinformationen direkt auszuwerten, insbesondere wenn diese wie obig erläutert als näherungsweise proportional zu entsprechenden (Massen-Dichteinformationen angenommen werden können. Dies erfolgt durch die folgenden Schritte:

- Erfassen von Bilddaten mit einer Tiefeninformation des Patienten (Schritt 101);
- Erzeugen eines Oberflächenmodells O des Patienten (Schritt 102) ;
- Ermitteln von (Massen-) Dichteinformationen $L\rho$ aus einer Röntgenmessung von zumindest einem Teil des Patienten (Schritt 103); und
- Ermitteln eines Patienten-Gesamtgewichts und/oder eines Body-Mass-Index des Patienten unter Verwendung des Oberflächenmodells O und der partiellen Dichteinformation Lp (Schritt 104).

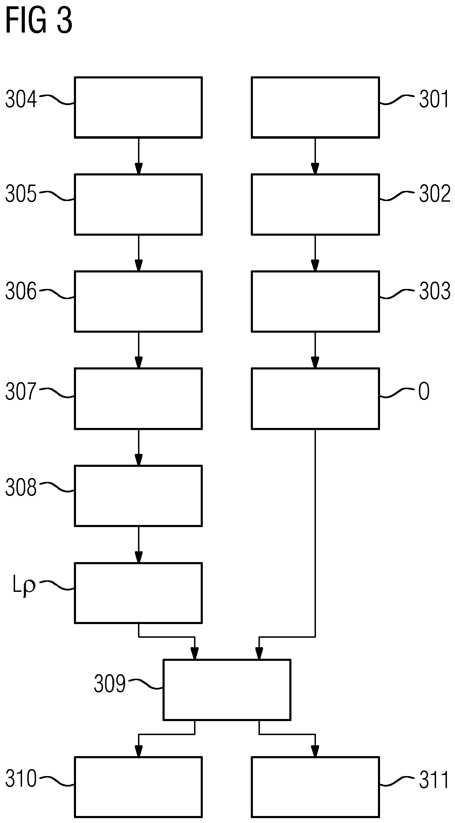

Eine Möglichkeit zur Ausgestaltung der einzelnen Schritte 101 bis 104 wird später noch mit Bezug auf Fig. 3 genauer erläutert werden.

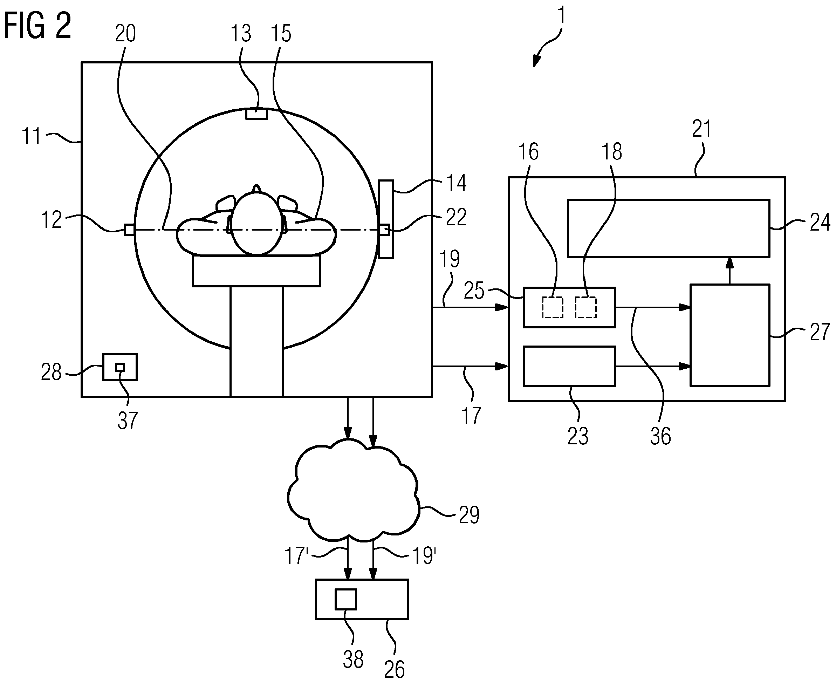

Eine beispielhafte Ausführungsform der Vorrichtung 1 der Erfindung ist in Fig. 2 schematisch dargestellt. In einem CT-Gerät 11 können über eine 3D Kamera 13 Bilddaten mit einer Tiefeninformation eines Patienten 15 erfasst werden. Über eine Schnittstelle 17 werden diese Bilddaten an eine an eine Auswerteeinheit 21 des CT-Geräts 11 übermittelt. In einer Erzeugungseinheit 23 zum Erzeugen eines Oberflächenmodells O des Patienten wird das Oberflächenmodells O des Patienten erzeugt.

Ferner können durch das CT-Gerät 1 über eine Topogram-Aufnahme (Röntgen-) Projektionsmessdaten erfasst werden. Aus den Projektionsmessdaten können eine Massen-Dichteinformation Lp von zumindest einem Teil des Patienten ermittelt werden. Die Massen-Dichteinformation Lp von zumindest einem Teil des Patienten wird über eine Schnittstelle 19 an die Auswerteeinheit 21 des CT-Geräts 11 übermittelt. In diesem Fall kann diese Information unmittelbar über die Schnittstelle 36 an die Berechnungseinheit 27 bereitgestellt werden. Alternativ können auch direkt die Projektionsmessdaten von dem CT Gerät übermittelt werden und die Berechnung der Massen-Dichteinformation Lp kann in einer Massen-Dichteinformations-Bestimmungseinheit 25 in der Auswerteeinheit 21 erfolgen.

In der Auswerteeinheit 21 kann in einer Berechnungseinheit 27 das Patienten-Gesamtgewicht bzw. der Body-Mass-Index unter Verwendung des Oberflächenmodells O und der Massen-Dichteinformation Lp berechnet und an eine Ausgabeeinheit 24 ausgegeben werden. Die Ausgabeeinheit 27 kann ein Display aufweisen, sie kann aber auch lediglich als Datenschnittstelle zur Ausgabe bzw. Übertragung von Patienten-Gesamtgewichtsdaten ausgebildet sein.

Weitere Details der Vorrichtung 1 werden im Folgenden mit Bezug auf Fig. 3 erläutert, die ein detailliertes Ausführungsbeispiel eines Verfahrens zur Ermittlung eines Patienten-Gesamtgewichts und eines Body-Mass-Index des Patienten, wie es in größerer Allgemeinheit schon mit Bezug zu Fig. 1 diskutiert wurde, darstellt. Hierbei werden zunächst in Schritt 301 Bilddaten mit einer optischen 3D-Kamera 13 erfasst. Hierbei kann beispielsweise eine Stereokamera, ein Time-Of-Flight-Verfahren oder Ähnliches genutzt werden. Die resultierenden Bilddaten 302 umfassen somit neben Helligkeits- bzw. Farbwerten für die einzelnen Bildpunkte, also zweidimensionalen Bilddaten, Tiefeninformationen für jeden einzelnen Bildpunkt bzw. für Gruppen der Bildpunkte.

In Schritt 303 wird basierend auf den Bilddaten 302 ein Oberflächenmodell O des Patienten 15 ermittelt. Im einfachsten Fall kann dies durch Segmentieren des den Patienten 15 abbildenden Bildbereichs erfolgen, da die Bilddaten aufgrund der vorhandenen Tiefeninformation in diesem Bereich bereits die Oberfläche des Patienten 15 beschreiben. Im Rahmen der medizinischen Bildgebung hat es sich jedoch allgemein als besonders vorteilhaft herausgestellt, zur Erzeugung eines Oberflächenmodells O des Patienten 15 ein Verfahren zu nutzen, das im Rahmen eines Maschinenlernens trainiert wurde.

Parallel zu den Schritten 301 und 303 oder vor, nach oder zwischen diesen Schritten wird, wie im Folgenden detailliert erläutert werden wird, eine (Massen-)Dichteinformation Lp ermittelt, wie allgemein schon zu Schritt 103 in Fig. 1 erläutert wurde. Hierzu wird zunächst eine Röntgenmessung 304 des Patienten 15 durchgeführt. Hierzu wird die Röntgenquelle 12 aktiviert und die resultierende Röntgenstrahlung wird nach Durchtritt durch den Patienten 15 durch den Röntgendetektor 14 erfasst. Einzelne Detektorelemente 22 des Röntgendetektors 14 erfassen hierbei die Intensität eines Röntgenstrahls 20 nach Durchtritt durch den Patienten 15. Hierbei resultieren Röntgenprojektionsdaten 305, bei denen die Bilddaten der einzelnen Bildpunkte jeweils vom Linienintegral über den jeweiligen Röntgenstrahl 20, der dem dem Bildpunkt zugeordneten Detektorelement 22 zugeordnet ist, über die Schwächungen entlang dieses Strahls 20 entsprechen. Aus diesen Werten kann, wie im Folgenden genauer erläutert werden wird, eine Information über die Dichte des Patienten 15 im Bereich dieses Strahls 20 ermittelt werden.

Prinzipiell könnten im Rahmen des Verfahrens auch mehrere Röntgenprojektionsaufnahmen erfasst werden, insbesondere um rekonstruierte Bilddaten eines CT-Scans zu ermitteln. Dies würde es ermöglichen, dreidimensionale Dichtinformationen bezüglich des Patienten 15 zu erhalten. Wird ein solcher dreidimensionaler 3D-Scan jedoch nicht ohnehin ausgeführt, bevor das Patienten-Gesamtgewicht bzw. der Body-Mass-Index ermittelt werden soll, würde hierdurch eine unnötig hohe Strahlenbelastung des Patienten 15 resultieren. Da jedoch typischerweise vor CT-Untersuchungen ohnehin eine Topogrammaufnahme ausgeführt wird, um die Untersuchung, insbesondere den zu erfassenden Bereich, exakt zu planen, kann das erfindungsgemäße Verfahren bei Nutzung von Röntgenprojektionsdaten ohne zusätzliche Strahlenbelastung für den Patienten und ohne zusätzliche erforderliche Messzeit durchgeführt werden. In Fällen, in denen ohnehin rekonstruierte Bilddaten eines CT-Scans ermittelt werden, beispielsweise wenn ein Maskendatensatz für eine CT-Angiographie ermittelt wird, bevor eine Kontrastmittelgabe erfolgt, kann durch Auswertung von dreidimensionalen rekonstruierten Bilddaten jedoch eine genauere Information über die Dichteverteilung im Patienten erreicht werden und somit potentiell die Genauigkeit eines ermittelten Patienten-Gesamtgewichts bzw. eines Body-Mass-Index weiter erhöht werden.

In Schritt 306 wird zunächst eine Strahlaufhärtungskorrektur für die Röntgenprojektionsdaten 305 durchgeführt, um korrigierte Röntgenprojektionsdaten 307 zu ermitteln. Wie bereits vorangehend erläutert, kann dies erfolgen, indem eine Funktion, insbesondere ein Polynom, auf die einzelnen Datenpunkte der Röntgenprojektionsdaten angewandt wird.

In Schritt 308 wird anschließend unter der Annahme, dass im gesamten Patienten das gleiche Material durchstrahlt wird, wobei insbesondere eine Durchstrahlung von Wasser angenommen wird, für jeden Bildpunkt der Röntgenprojektionsdaten eine integrierte Dichte als (Massen-)Dichteinformation $L_p$ berechnet. Wie bereits vorangehend erläutert wurde, kann anhand eines bekannten Absorptionskoeffizienten von Wasser für das genutzte Spektrum von Röntgenstrahlung und der Dichte von Wasser ein Proportionalitätsfaktor bestimmt werden, um dieses Linienintegral der Dichte aus den korrigierten Röntgenprojektionsdaten zu ermitteln.

Um das beschriebene Vorgehen umzusetzen, kann die Massen-Dichteinformations-Bestimmungseinheit 25 ein Korrekturmittel 16 implementieren, um die Strahlungsaufhärtungskorrektur in Schritt 306 durchzuführen und/oder ein Dichteberechnungsmittel 18, um die (Massen-)Dichteinformation $L_p$ in Schritt 308 aus den korrigierten Röntgenprojektionsdaten 307 zu ermitteln.

Die (Massen-)Dichteinformationen $L_p$ sowie das Oberflächenmodell O werden einem Algorithmus 309 zugeführt, der, wie im Folgenden noch genauer erläutert werden wird, im Rahmen eines Maschinenlernens trainiert wurde, um als Ausgangsdaten das Patienten-Gesamtgewicht 310 sowie einen Body-Mass-Index 311 des Patienten 15 auszugeben. Alternativ wäre es selbstverständlich auch möglich, nur einen dieser Werte zu ermitteln. Die ermittelten Größen können beispielsweise an der Ausgabeeinheit 24 der Auswerteeinheit 21 ausgegeben werden. Alternativ oder ergänzend können sie auch für eine spätere Auswertung gespeichert werden und/oder genutzt werden, um weitere Einrichtung zu steuern, beispielsweise um eine Kontrastmittelgabe an den Patienten 15 zu steuern.

In den bisherigen Beispielen wurde davon ausgegangen, dass die erfassten Bilddaten 302 und Röntgenprojektionsdaten 305 unmittelbar an eine separat von dem CT-Gerät 11 ausgebildete Auswerteeinheit 21 bereitgestellt werden, die die gesamte weitere Ermittlung des Patienten-Gesamtgewichts 310 bzw. des Body-Mass-Index 311 ausführt. Alternativ könnten Teile dieser Ermittlung oder auch die gesamte Ermittlung auch durch eine Steuereinrichtung 28 durchgeführt werden, die beispielsweise in das CT-Gerät 11 integriert sein kann, wie schematisch in Fig. 2 dargestellt ist. Dies kann beispielsweise zweckmäßig sein, wenn unmittelbar eine Kontrastmittelgabe für den Patienten 15 gesteuert werden soll oder wenn ohnehin bereits eine Datenvorverarbeitung in dem CT-Gerät 11 stattfindet. Es wäre selbstverständlich auch möglich, die Auswerteeinheit 21 in das CT-Gerät 11 zu integrieren oder eine separat ausgeführte Steuereinrichtung 28, beispielsweise einen PC in einem Steuerraum, zu nutzen.

In einigen Fällen kann es auch vorteilhaft sein, die genannten Schritte zumindest teilweise beabstandet von dem CT-Gerät durchzuführen. Beispielsweise kann eine Rechenvorrichtung 26 verwendet werden, die über ein Netzwerk 29, beispielsweise das Internet, mit dem CT-Gerät 11 kommuniziert. Die Schnittstellen 17', 19' der Rechenvorrichtung 26 können in diesem Fall Netzwerkschnittstellen sein oder über eine gemeinsame Netzwerkschnittstelle ansprechbar sein. Die Rechenvorrichtung 26 kann als einzelner Server ausgebildet sein, es kann sich jedoch auch um eine Cloudlösung handeln, bei der die beschriebene Funktionalität gemeinsam durch eine Vielzahl von Recheneinrichtungen 26 bereitgestellt wird. In der Steuereinrichtung 28 bzw. der Rechenvorrichtung 26 können die beschriebenen Abläufe beispielsweise implementiert werden, indem ein entsprechendes Computerprogramm in die jeweilige Speichereinrichtung 37, 38 geladen und ausgeführt wird.

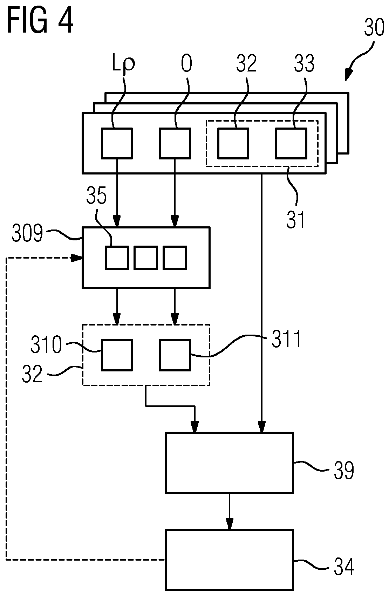

Fig. 4 zeigt schematisch eine Möglichkeit, um den Algorithmus 309 im Rahmen eines Maschinenlernens zu trainieren. Der Algorithmus 309 weist hierbei eine Vielzahl von Parametern 35 auf, deren Werte durch das im Folgenden beschriebene Lernverfahren festgelegt werden. Bei den Parametern 35 kann es sich beispielsweise um Eingangsgewichte der künstlichen Neuronen eines künstlichen neuronalen Netzes handeln.

Um den Algorithmus 309 zu trainieren bzw. die Parameter 35 zu wählen, werden zunächst eine Vielzahl von Trainingsdatensätzen 30 bereitgestellt. Die Trainingsdatensätze 30 umfassen jeweils für einen Patienten die (Massen-)Dichteinformation $L_p$ sowie das Oberflächenmodell O, die wie vorangehend erläutert ermittelt werden können. Zusätzlich umfasst jeder der Trainingsdatensätze 30 Sollwerte 31 für das Patienten-Gesamtgewicht 310 und den Body-Mass-Index 311. Diese Sollwerte 31 sind die Größen, die bei einem optimal trainierten Algorithmus 309 für den jeweiligen Trainingsdatensatz 30 ermittelt werden sollten. Der Sollwert 32 für das Patienten-Gesamtgewicht 310 kann beispielsweise durch ein Wiegen des Patienten ermittelt werden. Ist somit das Gewicht des Patienten bekannt, kann der Sollwert 33 für den Body-Mass-Index wie üblich berechnet werden, in dem dieses Gewicht durch das Quadrat der separat gemessenen Größe des Patienten 15 geteilt wird.

Die Parameter 35 des Algorithmus 309 werden zunächst zufällig oder auf andere Weise initialisiert. Anschließend wird für einen Teil der Trainingsdatensätze 30 jeweils die (Massen-)Dichteinformation Lp und das Oberflächenmodell O dem Algorithmus 309 als Eingangsdaten zugeführt, um für den jeweiligen Trainingsdatensatz Ausgangsdaten 32 für das Patienten-Gesamtgewicht 310 und den Body-Mass-Index 311 zu ermitteln.

Anschließend wird eine Kostenfunktion 39 ausgewertet, deren Wert von der Abweichung der Ausgangswerte 32 von den Sollwerten 31 abhängt. In Abhängigkeit der Kostenfunktion 39 erfolgt eine Fehlerrückführung 34, um die Parameter 35 anzupassen und somit durch mehrmaliges Wiederholen des beschriebenen Vorgehens die Kostenfunktion 39 zu minimieren.

Eine schnellere Konvergenz kann beispielsweise erreicht werden, wenn der Algorithmus 309 differenzierbar ist. In diesem Fall kann im Rahmen der Fehlerrückführung 34 die Ableitung der Kostenfunktion ausgewertet werden, um zu ermitteln, wie stark die Parameter 35 in welche Richtung angepasst werden sollten. Ein derartiges Vorgehen zur Fehlerrückführung ist insbesondere im Bereich der neuronalen Netze im Stand der Technik gut bekannt und soll daher nicht detailliert erläutert werden.

Soll eine Ermittlung direkt anhand einer Röntgenschwächungsinformation erfolgen, so kann hierfür insbesondere das erläuterte Training derart modifiziert werden, dass die Trainingsdatensätze statt der jeweiligen (Massen-)Dichteinformation eine Röntgenschwächungsinformation umfassen.

Allgemein kann das erfindungsgemäße Verfahren auch wie im Folgenden erläutert beschrieben werden. Hierbei wird zunächst ohne Einschränkung der Allgemeinheit ein Verfahren zur Bestimmung des Patientengewichts mit drei im Folgenden angegebenen Schritte vorgeschlagen:

1. Ermittlung eines Oberflächenmodells **O** des gesamten Patienten, z.B. mithilfe einer optischen 3D-Kamera. [Eine 3D-Kamera ist bei neueren CT-Systemen zum Zweck der Patientenpositionierung und Planung des Scans oder Topogramms vorhanden. Das Patientenmodell (Avatar), das insbesondere ein Oberflächenmodell umfasst, wird beispielsweise durch

Machine Learning-basierte Methoden beschafft.]

2. Ermittlung von (Massen-)Dichteinformation {Lρ} von zumindest einem Teil des Patienten mithilfe einer Röntgen-Projektionsmessung.
[Diese kann auch durch ein Topogramm in einem CT-System repräsentiert werden. Ein Topogramm, zumindest des zu untersuchenden Körperabschnitts, liegt beim regulären Ablauf einer CT-Untersuchung vor, um den eigentlichen Scanbereich exakt zu definieren, d.h. es muss keine zusätzliche Strahlung zum Zweck der Gewichtsbestimmung mithilfe des erfindungsgemäßen Verfahrens appliziert werden.]

3. Berechnung eines Patienten-Gesamtgewichts unter Verwendung des Oberflächenmodells **O** aus (1.) und der partiellen Dichteinformation {Lρ} aus (2.).

Die Massendichteinformation ist aus einer Röntgenmessung nicht direkt verfügbar, sondern nur die Signalschwächung entlang der gemessenen Strahlen bei der verwendeten Röntgenenergie. Die Massendichte muss deswegen abgeleitet werden. Im einfachsten Fall geschieht dies durch Mapping der Schwächungs-Linienintegrale auf Massendichte-Linienintegrale, vergleichbar mit dem Mapping zur Berechnung von Elektronendichten für die Strahlentherapieplanung. Eine konkrete Ausführungsform des Verfahrensschrittes (2.) sieht wie folgt aus: Messung von Signalabschwächungen bei einem bekannten Röntgenspektrum, was einer regulären Projektionsaufnahme bzw. einem Topogramm entspricht. Als Ergebnis liegen (im Allgemeinen polychromatische) Linienintegrale

$$L_{\mu} = -\ln\left(I/I_0\right) = -\ln\left(\int I(E)\exp\left(-\int \mu(E,\vec{r})d\vec{r}\right)dE \Big/ \int I(E)dE\right)$$

Für die gemessenen Strahlen vor.

Strahlaufhärtungskorrektur der Linienintegrale (= Linearisierung bzw. Monochromatisierung) durch eine Abbildung

$$L_{\mu}' = f\left(L_{\mu}\right),$$

um die Linienintegrale (approximativ) in der Form

$$L'_\mu \approx \int \mu'(\bar{r})d\bar{r} \quad (*)$$

darzustellen zu können. Dies ist ein bekannter Schritt in der CT-Rekonstruktion, der auf der Annahme basiert, dass alle im Körper vorkommenden Materialen in führender Ordnung wasserähnlich sind.

Die Abbildung f (x) ist dabei z.B. ein Polynom

$$f(x) = \sum_k a_k x^k$$

mit auf das Spektrum abgestimmten Koeffizienten, so dass (*) erfüllt ist.

(Approximative) Interpretation der unterschiedlichen Schwächungen als reine Dichteunterschiede, d.h.

$$L'_\mu \approx \int \mu'(\bar{r})d\bar{r} \approx \mu_{H_2O} \int \rho(\bar{r}) / \rho_{H_2O}(\bar{r})d\bar{r} = \mu_{H_2O} / \rho_{H_2O}(\bar{r}) \underbrace{\int \rho(\bar{r})d\bar{r}}_{=L_\rho} .$$

Als Ergebnis lassen sich Massendichte-Linienintegrale

$$L_\rho \approx L'_\mu \cdot \rho_{H_2O} / \mu_{H_2O} = \rho_{H_2O} / \mu_{H_2O} \cdot f(L_\mu)$$

angeben, bzw. korrekterweise ein Satz {Lp} aller gemessenen Strahlen.

Zu 3.: Die Berechnung des Patientengewichts erfolgt vorzugsweise mithilfe eines Machine Learning-basierten Ansatz (z.B. einem Convolutional Neural Network) unter Verwendung des Oberflächenmodells **O** und der Dichteinformation {Lρ} als Eingangsdaten und dem Patientengewicht als Ausgang. Dazu muss das Netzwerk mit einer Vielzahl von Datensätzen bestehend aus dem Input **O,** {Lρ} und mit einer bekanntem Patientengewicht (z.B. mit einer geeichten Waage bestimmt) trainiert werden.

Das vorgeschlagene Verfahren nutzt im regulären CT-Workflow ermittelte Informationen (3D Kamera und Topogramm), um ohne Beeinflussung/Störung des Untersuchungsablaufs zusätzliche relevante Patienteninformationen zu ermitteln. Es ist keine zusätzliche Hardware erforderlich, die die Gerätekosten erhöhen würden.

Gegenüber bestehenden oder naheliegenden Ideen, bei denen das mithilfe einer 3D-Kamera bestimmte Oberflächenmodell zur Schätzung des Patientengewichts genutzt wird, verwendet der vorgeschlagene Ansatz eine weitere Informationsquelle, die einen direkten Schluss auf die Dichteverteilung ermöglicht. Dies ist von Vorteil, da selbst bei identischer Körperoberfläche durch z.B. einen unterschiedlichen Fettanteil das Gewicht variieren kann.

Anstelle des Patientengewichts kann auch (oder zusätzlich) der Body-Mass-Index (BMI) ermittelt werden. Dazu wird lediglich der BMI als weiterer) Output im Training für den Machine Learning Algorithmus in Schritt (3.) verwendet.

Statt Projektionsaufnahmen können in Schritt (2.) auch die rekonstruierten Bilddaten eines nativen CT Scans verwendet werden. Dann liegen nicht nur Dichte-Linienintegrale, sondern sogar eine räumliche Dichteverteilung vor. In diesem Fall muss die zunächst rekonstruierte Schwächungsverteilung (CT-Werte) in Massendichtewerte transformiert werden. Die einfachste Realisierung stellt ein Mapping von CT-Werten auf Massedichte-Werte dar, bekannt aus der Strahlentherapieplanung. Alternativ können direkt Massendichte-Verteilungen mithilfe von Mehrmaterial-Zerlegungen rekonstruiert werden. Diese sind z.B. bekannt aus DE 102016209674A1.

Im Falle von Mehrenergie-Topogrammen bzw. -CT Aufnahmen, insbesondere mithilfe von Photon-zählenden Detektoren, gibt es zudem bekannte Verfahren, um auf Basis von Materialzerlegungen Massendichte-Linienintegrale bzw. räumliche Massedichte-Verteilungen zu berechnen [R. E. Alvarez and A. Macovski, "Energy-selective reconstructions in X-ray computerized tomography," Phys.Med.Biol., 21 (5), pp.733-744, 1976], [Williamson et.al., "On two-parametermodels of photon cross sections: Application to dual-energy CT imaging," Med.Phys. 33 (11), pp.4115-4129, 2006].

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

**Patentansprüche**

1. Verfahren zum Bestimmen eines Patientengewichts und/oder eines Body-Mass-Index eines Patienten (15) aufweisend die Schritte:

   - Erfassen von Bilddaten (302) mit einer Tiefeninformation des Patienten (15),
   - Basierend auf den Bilddaten (302), Erzeugen eines Oberflächenmodells (**O**) des Patienten (15),
   - Ermitteln von einer (Massen-)Dichteinformation (L$\rho$), die aus einer Röntgenmessung (304) des Patienten (15) ermittelt wird, oder Röntgenschwächungsinformation von zumindest einem Teil des Patienten (15), und
   - Ermitteln eines Patienten-Gesamtgewichts (310) und/oder des Body-Mass-Index (311) des Patienten (15) unter Verwendung des Oberflächenmodells (**O**) und der, insbesondere partiellen, Dichteinformation (L$\rho$) oder Röntgenschwächungsinformation.

2. Verfahren nach Anspruch 1, **dadurch gekennzeichnet, dass** die Bilddaten (302) mit einer optischen 3D-Kamera (13) erfasst werden oder erfasst worden sind.

3. Verfahren nach Anspruch 1 oder 2, **dadurch gekennzeichnet, dass** die (Massen-)Dichteinformation (L$\rho$) aus Röntgenprojektionsdaten (305), insbesondere aus Röntgenprojektionsdaten (305) einer Topogrammaufnahme, oder aus rekonstruierten Bilddaten eines nativen CT Scans ermittelt wird.

4. Verfahren nach Anspruch 3, **dadurch gekennzeichnet, dass** die Röntgenprojektionsdaten (305) durch eine Strahlaufhärtungskorrektur (306) korrigiert werden, wobei die (Massen-)Dichteinformation (L$\rho$) aus den derart korrigierten Röntgenprojektionsdaten (307) ermittelt wird.

5. Verfahren nach Anspruch 3 oder 4, **dadurch gekennzeichnet, dass** aus den Röntgenprojektionsdaten (305) oder den korrigierten Röntgenprojektionsdaten (307) unter der Annahme gleichen durchstrahlten Materials, wobei insbesondere eine Durchstrahlung von Wasser angenommen wird, für jeden Bildpunkt der Röntgenprojektionsdaten (305) eine integrierte Dichte als (Massen-)Dichteinformation (L$\rho$) berechnet wird.

6. Verfahren nach einem der vorangehenden Ansprüche, wobei der Schritt des Ermittelns unter Anwendung eines Machine-Learning Verfahrens, insbesondere unter Anwendung eines trainierten Algorithmus (309), erfolgt.

7. Verfahren nach einem der vorangehenden Ansprüche, **dadurch gekennzeichnet, dass** die Ermittlung des Patienten-Gesamtgewichts (310) und/oder des Body-Mass-Index (311) mithilfe eines Machine-Learning basierten Ansatzes unter Verwendung des Oberflächenmodells (**O**) und der (Massen-)Dichteinformation (L$\rho$) oder Röntgenschwächungsinformation als Eingangsdaten erfolgt.

8. Vorrichtung zum Bestimmen eines Patientengewichts und/oder eines Body-Mass-Index eines Patienten, aufweisend

   - eine Schnittstelle (17, 17') zum Erfassen von Bilddaten (302) mit einer Tiefeninformation des Patienten (15) ;
   - eine Erzeugungseinheit (23) zum Erzeugen eines Oberflächenmodells (**0**) des Patienten (15) basierend auf den Bilddaten (302);
   - eine Schnittstelle (36) zum Erfassen von einer Röntgenmessung oder Röntgenschwächungsinformation von zumindest einem Teil des Patienten (15),
   - eine Berechnungseinheit (27) zum Ermitteln eines Patienten-Gesamtgewichts (310) und/oder des Body-Mass-Index (311) des Patienten (15) unter Verwendung des Oberflächenmodells (**O**) und einer durch eine Massen-Dichteinformations-Bestimmungseinheit (25) aus der Röntgenmessung ermittelten (Massen-)Dichteinformation (L$\rho$) oder der Röntgenschwächungsinformation von zumindest einem Teil des Patienten (15), und
   - eine Ausgabeeinheit (24) zum Ausgeben des ermittelten Patientengewichts (310) und/oder des Body-Mass-Index (311) des Patienten (15).

9. Computerprogrammprodukt mit einem Computerprogramm, welches direkt in eine Speichereinrichtung (37, 38) einer Steuereinrichtung (28) einer Vorrichtung nach Anspruch oder einer mit einem CT-Gerät und einer Einheit zum Erfassen von Bilddaten mit einer Tiefeninformation des Patienten in Verbindung stehenden Rechenvorrichtung (26) ladbar ist, mit Programmabschnitten, um alle Schritte des Verfahrens nach einem der vorangehenden Ansprüche auszuführen, wenn das Computerprogramm in der Steuereinrichtung (28) oder der Rechenvorrichtung (26) ausgeführt wird.

10. Computerlesbares Medium, auf welchem von einer mit einem CT-Gerät und einer Einheit zum Erfassen von Bilddaten mit einer Tiefeninformation des Patienten in Verbindung stehenden Rechnereinheit einlesbare und ausführbare Programmabschnitte gespeichert sind, um alle Schritte des Verfahrens nach einem der Ansprüche 1 bis 7 auszuführen, wenn die Programmabschnitte von der Rechnereinheit ausgeführt werden.

**Claims**

1. Method for determining a patient weight and/or a body mass index of a patient (15), having the steps:

   - acquiring image data (302) containing depth information of the patient (15),
   - generating a surface model **(O)** of the patient (15) on the basis of the image data (302),
   - determining (mass) density information ($L\rho$) which is determined from an X-ray measurement (304) of the patient (15) or X-ray attenuation information of at least part of the patient (15), and
   - determining a total patient weight (310) and/or the body mass index (311) of the patient (15) using the surface model **(O)** and the in particular partial density information ($L\rho$) or X-ray attenuation information.

2. Method according to claim 1, **characterised in that** the image data (302) is or has been acquired using an optical 3D camera (13).

3. Method according to claim 1 or 2, **characterised in that** the (mass) density information ($L\rho$) is determined from X-ray projection data (305), in particular from X-ray projection data (305) of a topogram image, or from reconstructed image data of a native CT scan.

4. Method according to claim 3, **characterised in that** the X-ray projection data (305) is corrected by beam hardening correction (306), wherein the (mass) density information ($L\rho$) is determined from the thus corrected X-ray projection data (307).

5. Method according to claim 3 or 4, **characterised in that**, for each pixel of the X-ray projection data (305), an integrated density is calculated as (mass) density information ($L\rho$) from the X-ray projection data (305) or the corrected X-ray projection data (307) on the assumption of a same irradiated material, wherein, in particular, irradiation of water is assumed.

6. Method according to one of the preceding claims, wherein the determining step is carried out using a machine learning method, in particular using a trained algorithm (309) .

7. Method according to one of the preceding claims, **characterised in that** the determining of the total patient weight (310) and/or body mass index (311) takes place using a machine learning based approach using the surface model **(O)** and the (mass) density information ($L\rho$) or X-ray attenuation information as input data.

8. Apparatus for determining a patient weight and/or a body mass index of a patient, having

   - an interface (17, 17') for acquiring image data (302) containing depth information of the patient (15);
   - a generating unit (23) for generating a surface model **(O)** of the patient (15) based on the image data (302);
   - an interface (36) for acquiring an X-ray measurement or X-ray attenuation information of at least part of the patient (15),
   - a calculating unit (27) for determining a total patient weight (310) and/or the body mass index (311) of the patient (15) using the surface model **(O)** and the (mass) density information ($L\rho$) or X-ray attenuation information of at least part of the patient (15) determined from the X-ray measurement by means of a mass density information determining unit (25), and
   - an output unit (24) for outputting the determined patient weight (310) and/or the body mass index (311) of the patient (15).

9. Computer program product comprising a computer program which can be loaded directly into a storage device (37, 38) of a control device (28) of an apparatus according to claim 8 or of a computing apparatus (26) connected with a CT device and a unit for acquiring image data containing depth information of the patient, having program sections for carrying out all the steps of the method according to one of the preceding claims when the computer program is executed in the control device (28) or the computing apparatus (26).

**10.** Computer-readable medium on which are stored program sections that can be read and executed by a processing unit connected with a CT device and a unit for acquiring image data containing depth information of the patient in order to carry out all the steps of the method according to one of claims 1 to 7 when the program sections are executed by the processing unit.

**Revendications**

**1.** Procédé de détermination du poids d'un patient et/ou de l'indice de masse corporelle d'un patient (15) comportant les stades :

- saisie de données (302) d'image ayant une information de profondeur du patient (15),
- sur la base des données (302) d'image, production d'un modèle (O) de surface du patient (15),
- détermination d'une information (Lp) de (masse) densité, qui est déterminée à partir d'une mesure (304) aux rayons X du patient (15), ou d'une information d'atténuation des rayons X d'au moins une partie du patient (15), et
- détermination d'un poids (310) total du patient et/ou de l'indice (311) de masse corporelle du patient (15) en utilisant le modèle (O) de surface et l'information (Lp), notamment partielle, de densité ou l'information d'atténuation des rayons X.

**2.** Procédé suivant la revendication 1, **caractérisé en ce que** les données (302) d'image sont saisies ou ont été saisies par une caméra (13) optique en 3D.

**3.** Procédé suivant la revendication 1 ou 2, **caractérisé en ce que** l'on détermine l'information (Lp) de (masse) densité à partir de données (305) de projection de rayons X, notamment à partir de données (305) de projection de rayons X d'un enregistrement de topogramme, ou à partir de données d'image reconstruites d'un scan CT natif.

**4.** Procédé suivant la revendication 3, **caractérisé en ce que** l'on corrige les données (305) de projection de rayons X par une correction (306) de durcissement du rayonnement, dans lequel on détermine l'information (Lp) de (masse) densité à partir des données (307) de projection de rayons X ainsi corrigées.

**5.** Procédé suivant la revendication 3 ou 4, **caractérisé en ce qu'**à partir des données (305) de projection de rayons X ou des données (307) de projection de rayons X corrigées, en supposant que les matériaux traversés sont pareils, notamment en supposant que l'on traverse de l'eau, on calcule, pour chaque point image des données (305) de projection de rayons X, comme information (Lp) de (masse) densité, une densité intégrée.

**6.** Procédé suivant l'une des revendications précédentes, dans lequel le stade de détermination s'effectue en appliquant un procédé d'apprentissage automatique, notamment en appliquant un algorithme (309) ayant subi un apprentissage.

**7.** Procédé suivant l'une des revendications précédentes, **caractérisé en ce que** la détermination du poids (310) total du patient et/ou de l'indice (311) de masse corporelle s'effectue à l'aide d'une opération reposant sur l'apprentissage automatique, en utilisant le modèle (O) de surface et l'information (Lp) de (masse) densité ou l'information d'atténuation des rayons X comme données d'entrée.

**8.** Installation de détermination du poids d'un patient et/ou de l'indice de masse corporelle d'un patient, comportant :

- une interface (17, 17') de saisie de données (302) d'image ayant une information de profondeur du patient (15) ;
- une unité (23) de production d'un modèle (O) de surface du patient (15) reposant sur les données (302) d'image ;
- une interface (36) de saisie d'une mesure aux rayons X ou d'une information d'atténuation des rayons X par au moins une partie du patient (15),
- une unité (27) de calcul pour la détermination d'un poids (310) total du patient et/ou de l'indice (311) de masse corporelle du patient (15) en utilisant le modèle (O) de surface et une information de (masse) densité obtenue par une unité (25) de détermination de l'information (Lp) de (masse) densité, à partir de la mesure aux rayons X ou de l'information d'atténuation des rayons X par au moins une partie du patient (15), et
- une unité (24) de sortie pour sortir le poids (310) du patient et/ou l'indice (311) de masse corporelle du patient (15), qui a été déterminé.

**9.** Produit de programme d'ordinateur, comprenant un programme d'ordinateur, qui peut être chargé directement dans un dispositif (37, 38) de mémoire d'un dispositif (28) de commande d'une installation suivant la revendication 8 ou d'un système (26) informatique en liaison avec un appareil CT et avec une unité de détection de données d'image ayant une information de profondeur du patient, comportant des parties de programme pour exécuter tous les stades du procédé suivant l'une des revendications précédentes, lorsque le programme d'ordinateur est réalisé dans le dispositif (28) de commande ou dans le système (26) informatique.

10. Support, déchiffrable par ordinateur, sur lequel sont mises en mémoire des parties de programme pouvant être exécutées et déchiffrables par une unité informatique en liaison avec un appareil CT et une unité de saisie de données d'image ayant une information de profondeur du patient, pour effectuer tous les stades du procédé suivant l'une des revendications 1 à 7, lorsque les parties du programme sont réalisées par l'unité informatique.

FIG 1

101

102

103

104

FIG 2

# FIG 3

FIG 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2016262714 A1
- DE 10201609674 A1
- DE 102016209674 A1

**In der Beschreibung aufgeführte Nicht-Patentliteratur**

- **R. E. ALVAREZ ; A. MACOVSKI.** Energy-selective reconstructions in X-ray computerized tomography. *Phys.Med.Biol.,* 1976, vol. 21 (5), 733-744
- **WILLIAMSON.** On two-parametermodels of photon cross sections: Application to dual-energy CT imaging. *Med.Phys.,* 2006, vol. 33 (11), 4115-4129
- **WILLIAMSON.** On two-parametermodels of photon cross sections: Application to dual-energy CT imaging. *Med.Phys,* 2006, vol. 33 (11), 4115-4129